# EUROPEAN PATENT SPECIFICATION

(11) **EP 2 570 139 B1**
(45) Date of publication and mention of the grant of the patent: **07.08.2019**
(21) Application number: 11780621.6
(22) Date of filing: 10.05.2011
(51) Int. Cl.: A61L 27/00, A61L 27/38, C12N 5/071

(54) **METHOD FOR PRODUCING CELL SHEET**
VERFAHREN ZUR HERSTELLUNG EINER ZELLFOLIE
PROCÉDÉ DE PRODUCTION D'UN FEUILLET CELLULAIRE

(30) Priority: 10.05.2010 JP 2010108674
(43) Date of publication of application: 20.03.2013
(73) Proprietor: Yasukawa, Tsutomu, Aichi 464-0833 (JP)
(72) Inventor: TAKAHASHI, Masayo, Kobe-shi Hyogo 650-0047 (JP); YASUKAWA, Tsutomu, Nagoya-shi Aichi 467-8601 (JP)
(74) Representative: J A Kemp
(86) International application number: PCT/JP2011/060778
(87) International publication number: WO 2011/142364

(56) References cited:
- WO-A1-2009/127809
- JP-A- H09 501 303
- JP-A- 2006 501 848
- JP-A- 2007 509 643
- KUBOTA A ET AL: "Transplantable retinal pigment epithelial cell sheets for tissue engineering", BIOMATERIALS, ELSEVIER SCIENCE PUBLISHERS BV., BARKING, GB, vol. 27, no. 19, 1 July 2006 (2006-07-01), pages 3639-3644, XP027950958, ISSN: 0142-9612 [retrieved on 2006-07-01]
- KUBOTA, A. ET AL.: 'Transplantable retinal pigment epithelial cell sheets for tissue engineering' BIOMATERIALS vol. 27, no. 19, 2006, pages 3639 - 3644, XP027950958
- YAJI, N. ET AL.: 'Transplantation of tissue- engineered retinal pigment epithelial cell sheets in a rabbit model' BIOMATERIALS vol. 30, no. 5, 2009, pages 797 - 803, XP025801472
- NICOLAISSEN, B. ET AL.: 'Behavior of human RPE cultured on Bruch's membrane and on necrotic debris' INVEST OPHTHALMOL VIS SCI vol. 30, no. 5, 1989, pages 813 - 822, XP055098426
- RISA NONAKA: 'A role of fibulin-5 in the elastic fiber formation' THE ANNUAL REPORT OF THE HOSHI COLLEGE OF PHARMACY no. 51, 2009, pages 17 - 22, XP008168230

## Description

### Technical Field

The present invention relates to a method for producing a cell sheet using a plane substrate, particularly a method for producing a retinal pigment epithelial sheet. The present invention also relates to a method for producing a Bruch's membrane using a plane substrate.

### Background Art

Age-related macular degeneration (AMD) is currently one of the major causative diseases of legal blindness in developed countries, and is mainly seen in elderly citizens of 50 years or older. Age-related macular degeneration is a disease caused by age-related changes of macula, and is largely classified into an exudative type and an atrophic type. Exudative age-related macular degeneration is a disease wherein, in elderly citizens, a new blood vessel is developed in macula from the choroid membrane, and bleeding and exudative lesion are produced under retinal pigment epithelium or under retina to finally form scar tissues. Atrophic age-related macular degeneration is a disease associated with atrophy of macular region and drusen accumulation. In addition, a precursor lesion before reaching an exudative type or atrophic type age-related macular degeneration is sometimes particularly called an early age-related macular degeneration, and this lesion is also considered one pathology of the age-related macular degeneration.

For the treatment of age-related macular degeneration in the case of a mild exudative type, a treatment method aiming at degeneration/removal of new blood vessel by a surgical therapy such as photodynamic therapy, laser photocoagulation, surgery of new blood vessel removal and the like, a drug therapy such as administration of an inhibitor of vascular endothelial growth factor (VEGF) relating to angiogenesis and the like can be selected. However, in the case of an exudative type or atrophic type which has progressed to show severe atrophy and deficiency of retinal pigment epithelium (RPE), the aforementioned means fails to show effectiveness. In such case, transplantation of retinal pigment epithelial cells or retinal pigment epithelium to a subretinal retinal pigment epithelium lost site is an effective treatment method (patent documents 1, 2).

When cells are used for transplantation, examples of the retinal pigment epithelial cells include retinal pigment epithelial cells separated from an eye of a fetus to be the donor and cultured, iris pigment epithelial cells separated from the patient's own eye and cultured, retinal pigment epithelial cells collected by scraping during surgery, and the like. However, when cells of other person are used as a donor, rejection is feared on the side of the patients, and even when the patient's own cells are used for the treatment, use of a different cell type for transplantation is not preferable. Furthermore, transplantation of separated and cultured cells shows a problematic survival rate, which prevents exertion of sufficient function as the epithelium. Therefore, a tissue consisting of retinal pigment epithelium-Bruch's membrane-choroid membrane and a retinal pigment epithelial sheet cultured on an artificial sheet have also been proposed as retinal pigment epithelium for transplantation (patent documents 1, 3). However, excised choroid membrane and artificial sheet are feared to form a blockade for epithelium engrafting and maintenance of function of the body. In recent years, moreover, transplantation of retinal pigment epithelial cells (non-patent document 1) or artificial sheet using iPS cells has been studied, and a solution to the problem of antigenicity has been sought. However, the problem of blockade for cell engraftment and maintenance of the function of the sheet in the body has yet to be solved, and the production of retinal pigment epithelium which is therapeutically effective for age-related macular degeneration and convenient to prepare, has been the problem.

Kubota et al (2006) Biomaterials 27:3639-3644 discloses transplantable retinal pigment epithelial cell sheets for tissue engineering. International patent publication WO2009/127809 A1 discloses a membrane for supporting cells, and in particular, retinal pigmented epithelial cells.

### [Document List]

### [patent documents]

patent document 1: JP-A- H9-501303
patent document 2: JP-A-2008-173333
patent document 3: JP-A-2007-509643

### [non-patent document]

non-patent document 1: Neuroscience Letters, 458, 2009, 126-131

### SUMMARY OF THE INVENTION

### Problems to be Solved by the Invention

The problem of the present invention is to develop a new method for simply producing a mammal-derived cell sheet without using an artificial sheet, and to provide the cell sheet to a subject in need of tissue transplantation. Particularly, the problem is to provide epithelium for transplantation, which shows high survival rate and is superior in function, for age-related macular degeneration accompanied by retinal pigment epithelial defect.

### Means of Solving the Problems

Generally, when epithelial cells containing retinal pigment epithelial cells are cultured in a low density on a flat dish, the basal part of the cells is formed on the contact surface side with the dish. The present inventors have found that, when retinal pigment epithelial cells separated from a human eye and cultured are three-dimensionally aggregated and cultured rather than on a plane substrate, that is, spherically (spheroidally) cultured, the cells present inside the spherical cell aggregate are killed by apoptosis, and Bruch's membrane is formed on cell aggregate surface to be in contact with the serum (unpublished data). They have also found that the cell surface differentiated as retinal pigment epithelium. Therefrom the inventors have studied the possibility of epithelium differentiation without induction of cell dedifferentiation, and formation of Bruch's membrane on the serum contact surface, under an environment where cell-cell adhesion preferentially occurs than adhesion with extracellular matrix even by normal plane culture, and found that Bruch's membrane is produced on the side opposite from normal, namely, the contact surface side with the serum, by plane culture of retinal pigment epithelial cells under high density conditions, that is at 10,000 cells/mm² - 120,000 cells/mm². They have also found that retinal pigment epithelial cells do not dedifferentiate but differentiate as epithelium by such culture. The present inventors have conducted intensive studies and completed the present invention. Accordingly, the present invention provides
[1] a method for producing a cell sheet, comprising the following steps;
   (1) a step of preparing a retinal pigment epithelial cell,
   (2) a step of seeding the prepared cell on a plane substrate at 10,000 cells/mm² - 120,000 cells/mm², and culturing the cell, wherein the cell sheet comprises a basement membrane formed on the side opposite from the side to be in contact with the plane substrate, and
   (3) a step of confirming formation of Bruch's membrane on the side opposite from the side to be in contact with the plane substrate of the cell;
[2] the method of the above-mentioned [1], wherein the obtained cell sheet comprises a tight junction formed between cells;
[3] the method of the above-mentioned [1], further comprising the following step (4):
   (4) a step of confirming the presence or absence of expression of a differentiation marker in the cultured cells obtained in step (2) ;
[4] the method of the above-mentioned [3], wherein, in step (4), the differentiation marker is at least one kind selected from the group consisting of bestrophin-1, RPE-65, cytokeratin, occludin, ZO-1, elastin, actin, type 1 collagen and type 4 collagen;
[5] a method for producing Bruch's membrane comprising a step of separating the Bruch's membrane formed by the method of any one of the above-mentioned [1] - [4];
[6] a cell sheet produced by the method of any one of the above-mentioned [1] - [4];
[7] a material for transplantation for treating a disease, comprising a cell sheet produced by the method of any one of the above-mentioned [1] - [4]; and
[8] Bruch's membrane produced by the method of the above-mentioned [5].

### Effect of the Invention

According to the present invention, a cell sheet possessing a layer structure reverse to that of a cell sheet obtained by a normal culture method, and having a constitution wherein the basement membrane is exposed can be prepared simply. Using the cell sheet of the present invention, the basement membrane can contact a transplantation target tissue without turning the sheet over. Therefore, the cell sheet can simplify the transplantation operation, is superior in the survival rate, and therefore, is extremely useful for transplantational use. For example, a retinal pigment epithelial cell sheet can be produced easily by plane culture, and can be applied to transplantation to patients with age-related macular degeneration. Particularly, when cells to be cultured are retinal pigment epithelial cells derived from iPS cells, rejection can be avoided in transplantation since the patient's own cells are utilized.

### Brief Description of the Drawings

Fig. 1 shows a photographed image of a sheet observed by seeding human retinal pigment epithelial cells at 5,000 cells/mm² on a 4-well Chamber Slide (Lab-Tek), wherein the arrow shows a shrunk cell sheet.
Fig. 2 shows a photographed image of a sheet observed by seeding human retinal pigment epithelial cells at 20,000 cells/mm² on a 4-well Chamber Slide (Lab-Tek), wherein shrinkage was not observed in the cell sheet.
Fig. 3 shows photographed images and stained images of cells when human retinal pigment epithelial cells were seeded at 20,000 cells/mm², wherein (A) is a photographed image of cells on day 0 after seeding, (B) is a photographed image of hard drusen on day 1 after seeding, (C) is a photographed image of cells on day 1 after seeding, (D) is a photographed image of Bruch's membrane on day 1 after seeding, (E) is a staining image of cell nuclei on day 1 after seeding, and (F) is a stained image of actin in Bruch's membrane on day 1 after seeding.
Fig. 4 is an elastin-stained image of reticulate elastic fiber after 2 weeks of plane culture of human retinal pigment epithelial cells.
Fig. 5 shows the results of Western blotting for a protein extracted from a human retinal pigment epithelial sheet, which confirm expression of each marker protein of RPE-65, occludin, cytokeratin-18, ZO-1, type 4 collagen and elastin.
Fig. 6 shows scanning electron microscopic images of a human retinal pigment epithelial sheet, wherein the arrows show collagen fiber and elastic fiber, and the bottom right figure shows exposed basal infolding (microvilli of basal part). Description of Embodiments

The present invention is explained in detail in the following. The present invention provides a method for producing a cell sheet, comprising the following steps;
(1) a step of preparing a retinal pigment epithelial cell, and
(2) a step of seeding the prepared cells on a plane substrate 10,000 cells/mm² - 120,000 cells/mm², and culturing the cells.

The cells prepared in step (1) may be from any mammal (e.g., human, monkey, mouse, rat, dog, cattle, horse, pig, sheep, goat, cat, rabbit, hamster, guinea pig etc.). Preferred are a human-derived cells.

As a cell type of the cells to be prepared, any of epithelial and endothelial type cells having adhesiveness are preferably used. Examples of such cells include retinal pigment epithelial cells.

The cells to be prepared may be the primary cells directly collected from a tissue or organ, or may be cells in several generations after passages. Furthermore, the cells may also be obtained by inducing differentiation of stem cells including embryonic stem cells (ES cells) which are undifferentiated cells, pluripotent stem cells such as mesenchymal stem cells having pluripotency and the like, and unipotent stem cells such as blood vessel endothelial progenitor cells having unipotency and the like. The ES cells may be ES cells produced by nuclear reprogramming of somatic cells. In addition, the cells to be used may be prepared by inducing differentiation of induced pluripotent stem cells (iPS cell) which have been reported in recent years. iPS cells are somatic cell-derived artificial stem cells having properties equivalent to those of ES cells, which can be produced by introducing a particular nuclear reprogramming substance (nucleic acid, protein, low-molecular-weight compound etc.) into somatic cells [Takahashi, K. and Yamanaka, S., Cell, 126: 663-676 (2006); Takahashi, K. et al., Cell, 131: 861-872 (2007)]. The condition/medium for differentiation of the aforementioned stem cells into desired differentiated cells may be conventionally known condition/medium, or may be appropriately determined by those of ordinary skill in the art. When the cell sheet to be produced by the present invention is used for transplantation, use of iPS cells is preferable since a cell sheet having no antigenicity to the transplantation subject can be obtained by using somatic cells of the transplantation subject as a source of the iPS cells.

The cells to be prepared in the present invention are preferably human retinal pigment epithelial cells. In addition, the retinal pigment epithelial cells are differentiated cells derived from stem cells or cells derived from an eye. The retinal pigment epithelial cells from an eye are collected by removing an eye from a dead body, quickly splitting the eye at the equatorial segment, removing the vitreous body and retina, and scraping the cells with a cell scraper or releasing the cells from Bruch's membrane with trypsin or EDTA solution. Thereafter, the cells are left standing in a culture medium to allow adhesion to the culture dish, induced to grow to a requisite amount of the cells, and the number of the cells is secured by appropriate subculture with trypsin treatment and the like. When differentiation of stem cells is induced, human ES cells or iPS cells are cultured in an ES cell differentiation medium added with Dkk-1 (Wnt antagonist) and Lefty A (Nodal antagonist). Rx, Pax6 and Mitf, which are retina progenitor cell markers, are expressed by culturing for a given period, and human retinal pigment epithelial cells can be obtained by confirming a polygonal form by morphological observation based on optical microscopic observation.

In step (2), a cell sheet can be produced by seeding the cells prepared above on a plane substrate in a high density, and culturing the cells. While the plane substrate in the present specification is not particularly limited as long as it is used for cell culture, for example, flask, flask for tissue culture, dish, petri dish, dish for tissue culture, multidish, microplate, microwell plate, multiplate, multiwell plate, Chamber Slide, schale, tube, tray, culture bag, roller bottle can be mentioned. Examples of the material of the plane substrate in the present specification include, but are not limited to, inorganic materials such as metal, glass, ceramic, silicon and the like, and organic materials such as elastomer, and plastic (e.g., polyester resin, polyethylene resin, polypropylene resin, ABS resin, nylon, acrylic resin, fluorine resin, polycarbonate resin, polyurethane resin, methylpentene resin, phenol resin, melamine resin, epoxy resin, vinyl chloride resin). While the plane substrate may be subjected to a surface treatment to improve the adhesiveness to adhesive cells, it may be free of any treatment so as to facilitate a step of detaching the cell sheet of the present invention from the plane substrate. When the plane substrate is treated, it may be a surface treatment with, for example, collagen, gelatin, Matrigel, poly-L-lysine, poly-D-lysine, laminin, fibronectin and the like.

The "high density" in the present specification means a density of the level not less than the cell density observed in a normal tissue from which the prepared cells derive. While the upper limit thereof can be appropriately determined by those of ordinary skill in the art, it is, for example, a cell density of the level at which the cells can be closely adhered to each other and are free of defective sheet formation and cell extinction due to excessive seeding. More specifically, "high density" is, for example, a cell density of 1- to 100-fold, preferably 1.2- to 50-fold, more preferably about 2.5-to 30-fold, particularly preferably about 5- to 10-fold, that observed in normal tissues. While the cell density observed in normal tissue varies depending on the kind of the tissue, it is, for example, about 3,000 cells/mm² in corneal endothelium and about 4,000 cells/mm² for retinal pigment epithelial cells.

The mammal-derived cells to be prepared in the present invention are retinal pigment epithelial cells. "High density" means "a density of the level not less than the cell density observed in a normal eye". Such density is concretely at least 4,000 cells/mm² or more. As shown in the following Examples, however, even when the density is not less than the cell density observed in a normal eye, when the cells are seeded on a plane substrate in a density not more than a given density, a contractile force acts on the formed cell sheet itself, and the area on seeding cannot be maintained. The inability to maintain the area on seeding is considered to be attributable to the facts that a certain number of cells are recruited to cover the serum contact surface of the retinal pigment epithelium, and cell density is inconsistent, as well as due to the survival rate of the cells themselves. However, even such shrunk sheet can be utilized for the below-mentioned use without any particular inconvenience. Therefore, the "density of the level not less than the cell density observed in a normal eye" is preferably about 5,000 cells/mm² or more, more preferably about 10,000 cells/mm² or more, when shrinkage of the formed cell sheet from the area on seeding is tolerable, and preferably about 20,000 cells/mm² or more when the formed cell sheet maintains the area on seeding. In addition, the upper limit thereof is a density that does not induce defective sheet formation and cell extinction due to excessive seeding. The "density of the level is preferably 10,000 cells/mm² - 120,000 cells/mm², particularly preferably 20,000 cells/mm² - 40,000 cells/mm².

A cell population in a single layer can be formed by culturing the cells seeded in the aforementioned high density in a culture medium. As the culture medium, any medium for cell culture can be used without any particular limitation as long as it is normally used in the art. For example, a basal medium described in "Tissue Culture Techniques, 3rd edition edited by Japanese Tissue Culture Association", page 581, Asakura Publishing Co., Ltd., such as F-10 medium, F12 medium, MEM, BME medium, DMEM, αMEM, IMD medium, ES medium, DM-160 medium, Fisher medium, WEe medium, RPMI1640 medium and the like, can be used depending on the kind of the cells to be used. Furthermore, serum (fetal bovine serum etc.), various growth factors, antibiotic, amino acid and the like may be added to the basal medium. The pH of the medium is preferably about 6 - about 8. Culture is generally performed at about 30 - about 40°C for about 15 - about 60 hr. Where necessary, aeration and agitation may also be performed.

In the cell sheet obtained by the method of the present invention, a tight junction is formed between cells, and a basement membrane is formed on the side opposite from the side to be in contact with the plane substrate. The side opposite from the side to be in contact with the plane substrate is a contact surface side between cells and serum. Formation of tight junction can be confirmed by observation of hexagonal, closely adhered cell form, and expression of intercellular occludin, ZO-1 and the like by immunostaining. Formation of Bruch's membrane including a basement membrane can be confirmed by observation of expression of elastin, type 1 collagen or type 4 collagen, and the like on the cell surface by immunostaining, and observation by a scanning electron microscope.

The mammal-derived cells to be prepared in the present invention are retinal pigment epithelial cells. The following step (4) may be further contained.
(4) a step of confirming the presence or absence of expression of a differentiation marker in the cultured cells obtained in step (2)

In step (4), completion of a cell sheet can be judged by confirming the presence or absence of expression of a differentiation marker in the cultured cells obtained in step (2). In the present specification, the differentiation marker may be expressed at any site of the cells (e.g., cytoplasm, cellular membrane, nuclear membrane and the like), and preferably, a marker expressed on the contact surface side between the seeded cells and serum is the target.

The "differentiation marker" in the present specification includes a transcriptional product, a translational product and a degradation product thereof, of a gene specifically expressed in differentiated cells, or showing amplified or attenuated expression as compared to other differentiated cells, undifferentiated cells or progenitor cells. Examples of such gene include neuron differentiation markers such as tubulin (particularly β tubulin), MAP2, neurofilament and neuron specific enolase, adipocyte differentiation markers such as aP2, glycerophosphate dehydrogenase, adipsin and leptin, osteoblast differentiation markers such as procollagen 1α1, RUNX2, alkaline phosphatase, osteopontin and osteocalcin, and retinal pigment epithelial differentiation markers such as bestrophin-1 (VMD2), RPE-65, cytokeratin, occludin, ZO-1, elastin, actin, type 1 collagen or type 4 collagen and the like.

A sample to be used for the "confirmation of the presence or absence of expression of a differentiation marker" is not particularly limited as long as it includes differentiation markers (e.g., RNA, protein, degradation product thereof and the like) derived from the cells cultured in step (2).

When the above-mentioned sample is RNA, the expression of a differentiation marker gene can be examined by preparing an RNA (e.g., total RNA, mRNA) fraction from the cells cultured in step (2) and detecting a transcriptional product of the marker gene contained in the fraction, or directly detecting a marker gene product in the cells without extracting RNA from the cells.

When RNA (e.g., total RNA, mRNA) fraction is prepared from cells, the preparation of the RNA fraction can be carried out using a known technique such as a guanidine-CsCl ultracentrifugation method, an AGPC method or the like, but total RNA of high purity can be prepared rapidly and conveniently from a trace amount of sample, using a commercially available RNA extraction kit (for example, RNeasy Mini Kit manufactured by QIAGEN, etc.). As the means for detecting the transcription products of the differentiation marker genes in the RNA fraction, for example, a method of using hybridization (Northern blotting, dot blotting, DNA chip analysis, etc.), a method of using PCR (RT-PCR, competitive PCR, real time PCR, etc.), or the like may be mentioned. From the viewpoint that the fluctuation in expression of differentiation marker genes can be detected from a trace amount of sample rapidly and conveniently with good quantitative capability, a quantitative PCR method such as competitive PCR, real time PCR or the like is preferred, while from the viewpoint that the fluctuation in the expression of a plurality of marker genes can be collectively detected, and the quantitative capability can be improved by the selection of the detection method, DNA chip analysis is preferred.

In the case of performing Northern blot or dot blot hybridization, detection of a differentiation marker gene can be performed using a nucleic acid (probe) which can be hybridized with the transcription product of the gene. Such nucleic acid may be exemplified by a nucleic acid which can be hybridized with the transcription product of a differentiation marker gene under highly stringent conditions. As the "highly stringent conditions", a hybridization reaction at 45°C in 6xSSC (sodium chloride/sodium citrate) followed by washing once or more at 65°C in 0.2xSSC /0.1% SDS, or the like may be mentioned. Those ordinarily skilled in the art can easily adjust the conditions to desired stringency by appropriately altering the salt concentration in the hybridization solution, the temperature for the hybridization reaction, the probe concentration, the length of the probe, the number of mismatches, the duration of the hybridization reaction, the salt concentration of the washing solution, the temperature of the washing process, or the like. The nucleic acid may be a DNA or an RNA, or may also be a DNA/RNA chimera. Preferably, the nucleic acid may be a DNA.

The nucleic acid used as the probe may be double-stranded or single-stranded. In the case of being double-stranded, the nucleic acid may be a double-stranded DNA, a double-stranded RNA or a hybrid of DNA:RNA. In the case of a single-stranded nucleic acid, an antisense strand can be used. The length of the nucleic acid is not particularly limited as long as the nucleic acid can be specifically hybridized with a target nucleic acid, and for example, the length is about not less than 15 bases, and preferably about not less than 30 bases. The nucleic acid is preferably labeled by a labeling agent so as to enable detection/quantification of the target nucleic acid. As the labeling agent, for example, a radioisotope, an enzyme, a fluorescent material, a luminescent material or the like is used. The radioisotope may be exemplified by [³²P], [³H], [¹⁴C] or the like. The enzyme is preferably an enzyme which is stable and has high specific activity, and for example, β-galatosidase, β-glucosidase, alkaline phosphatase, peroxidase, malate dehydrogenase or the like is used. The fluorescent material may be exemplified by fluorescamine, fluorescein isothiocyanate or the like. The luminescent material may be exemplified by luminol, a luminol derivative, luciferin, lucigenin or the like. Furthermore, biotin-(strept)avidin may also be used for the binding between a probe and a labeling agent.

In the case of Northern hybridization, an RNA fraction prepared as described above is separated by gel electrophoresis, subsequently transferred to a membrane made of nitrocellulose, nylon, polyvinylidene fluoride or the like, and hybridized under the "highly stringent conditions" described above in a hybridization buffer solution containing a labeled probe prepared as described above, and then the amount of the label bound to the membrane is measured for each band by an appropriate method, whereby the expression level of each of the differentiation marker genes can be measured. In the case of dot blotting, a membrane spotted with an RNA fraction is subjected to a hybridization reaction (performed for each marker genes), and the amount of label of the spot is measured, whereby the expression level of each of the marker genes can be measured.

In the case of DNA chip analysis, for example, a cDNA to which an appropriate promoter such as T7 promoter has been introduced is synthesized from an RNA fraction prepared as described above by a reverse transcription reaction, and a cRNA is further synthesized using an RNA polymerase (here, a labeled cRNA is obtained using a mononucleotide labeled with biotin or the like, as a substrate). This labeled cRNA is subjected to a hybridization reaction by contacting the cRNA with a chip on which the probes are immobilized, and the amount of the label bound to each of the immobilized probes is measured, whereby the expression level of each of the differentiation marker genes can be measured. This method is advantageous in terms of rapidity and convenience, as the number of differentiation marker genes to be detected (therefore, probes being immobilized) increases.

On the other hand, when a marker gene is detected without extracting RNA from the cells, in situ hybridization can be used as a detection technique therefor. In this method, instead of extracting RNA from the cells, cells are fixed by treating the cells with a fixative, preferably precipitative fixative, for example, acetone, or briefly incubating the cells in a buffered formaldehyde solution. After fixation, the cells are embedded in paraffin to form a block, and a thin section is cut out and can be used as a sample. A well-prepared paraffin-embedded sample can be preserved at room temperature for many years. As nucleic acid to be used as a probe, one similar to the above-mentioned nucleic acids can be used. In situ hybridization can be preferably used in the present invention since expression of a differentiation marker can be directly confirmed on the serum contact surface of the cells.

Alternatively, expression of a differentiation marker gene in the cultured cells in step (2) can be confirmed by preparing a protein fraction from the cells, and detecting a translational product (i.e., marker protein) of the marker gene contained in the fraction, or directly detecting a translational product of the marker gene in the cells without extracting a protein from the cells. The detection of the marker proteins can be performed according to an immunological assay method (for example, ELISA, FIA, RIA, Western blotting, etc.), using antibodies to the respective proteins, or in the case of proteins exhibiting a measurable physiological activity, such as an enzyme or the like, the detection can be performed by measuring the physiological activity using known techniques for the respective marker proteins. Or else, the detection of marker proteins can also be performed using a mass spectroscopy method such as MALDI-TOFMS or the like.

Furthermore, the antibody to each of the marker proteins can be obtained according to conventionally used polyclonal antibody or monoclonal antibody production techniques, using as a sensitizing antigen, said marker proteins, said protein or a partial peptide thereof.

In applying the individual immunological assay methods to the examination method of the present invention, it is not necessary to establish particular conditions or carry out particular operations. An appropriate measurement system for the differentiation marker proteins can be constructed by giving technical considerations that are obvious to those skilled in the art, to the conditions and operations which are conventional in the respective methods. For details of these conventional technical means, reference may be made to review articles and monographs. For example, reference can be made to "Radioimmunoassay", edited by Hiroshi Irie (Kodansha, published in 1974), "Radioimmunoassay, Supplemental", edited by Hiroshi Irie (Kodansha, published in 1979), "Enzyme Immunoassay", edited by Eiji Ishikawa et al. (Igaku Shoin, published in 1978), "Enzyme Immunoassay" (2nd edition), edited by Eiji Ishikawa, et al. (Igaku Shoin, published in 1982), "Enzyme Immunoassay" (3rd edition) edited by Eiji Ishikawa, et al. (Igaku Shoin, published in 1987), "Methods In Enzymology", Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies)) (published by Academic Press), and the like.

The aforementioned method for producing a cell sheet, wherein the cells are retinal pigment epithelial cells, further contains the following step (3):
(3) a step of confirming formation of Bruch's membrane on the side opposite from the side to be in contact with the plane substrate of the cells

The "Bruch's membrane" in the present specification is a thin membrane between the retinal pigment epithelium and choroid membrane, and is a layer lining the retinal pigment epithelium. It is a cell-free structure mainly made of a collagen fiber, to which choroid membrane and pigment epithelial cells are adhered, and functions as a passage to deliver substances from the choroid membrane to the outer layer of the retina where no blood vessel is present. Single layer epithelium is very fragile and easily broken during transplantation operation and the like, cells are conventionally grown on a substitute sheet such as an artificial sheet, an amniotic membrane and the like. However, cell differentiation is insufficient, and the artificial sheet blocks biophysical phenomena in the transplantation site. In contrast, since Bruch's membrane containing the original basement membrane and elastic fiber produced by the cells themselves is formed in the present invention, the strength sufficient to stand a transplantation operation can be obtained. In addition, since the Bruch's membrane of the present invention is formed on the side to be in contact with the serum, the sheet can be easily detached from a culture dish, including the Bruch's membrane.

The present invention also provides, in the aforementioned production method of retinal pigment epithelial cell sheet, a method of producing Bruch's membrane in vitro, comprising separating the formed Bruch's membrane from the retinal pigment epithelial cell sheet. The Bruch's membrane formed on the retinal pigment epithelium can be detached and recovered from the sheet by treating with EDTA, or pipetting and suction operation on the sheet surface in a magnesium and calcium-free addition medium. In addition, the Bruch's membrane can also be detached and recovered by suctioning the culture medium to allow the membrane to be in contact with the air, and performing a dehydration action on a sheet surface by a treatment with diluted alcohol.

The present invention also relates to a cell sheet obtained according to the aforementioned method for producing a retinal pigment epithelial cell sheet. The cell sheet can be used for living organisms, for example, in various uses of screening use and toxicity test use. In addition, the cell sheet of the present invention can be preferably transplanted, as a transplantation material for disease treatment, into a subject in need of tissue transplantation. Particularly, the retinal pigment epithelial cell sheet of the present invention is preferable as a transplantation material for retinal treatment of patients with ophthalmic diseases. Examples of the ophthalmic disease include age-related macular degeneration disease, retinitis pigmentosa and the like. In addition, the retinal pigment epithelial cell sheet can also be utilized for various screening uses such as drug efficacy screening, toxicity evaluation and the like for the aforementioned ophthalmic diseases. For example, the cell sheet can be applied to the screening for a toxic substance according to the method described in JP-A-2007-517210. Furthermore, the retinal pigment epithelial cell sheet can also be utilized as a vitro model for the evaluation of various different functions possessed by retinal pigment epithelial cells in the body, such as functions involved in the maintenance of visual cells (e.g., capacity of phagocytosis of photoreceptor outer segment, neuroprotective action and the like), pumping action, retinal blood vessel barrier function by tight junction, and the like.

The transplantation material for disease treatment of the present invention can be used for treating the above-mentioned diseases in human and mammals other than human (e.g., monkey, mouse, rat, dog, cattle, horse, pig, sheep, goat, cat, rabbit, hamster, guinea pig etc.).

While the area range of lesion to which the transplantation material for disease treatment of the present invention is applicable may vary depending on the target disease, animal species of the administration subject, age, sex, body weight, symptom and the like, when it is applied to, for example, an age-related macular degeneration disease, the area range of lesion for transplantation is generally 0.07 cm² - 0.28 cm².

The transplantation material for disease treatment of the present invention may be transplanted at once or in several portions. The number of transplantation operations is determined according to the disease, by healthcare professionals and based on the guideline. For example, when the disease is an age-related macular degeneration disease, the retinal pigment epithelial cell sheet of the present invention may be transplanted two times or more according to the severity of the disease. When the transplantation is performed plural times, the interval therebetween is not particularly limited and may be performed after lapse of several days to several weeks.

The transplantation material for disease treatment of the present invention is transplanted according to an appropriate transplantation method by healthcare professionals in line with the guideline. For example an endoscopic technique can be adopted. In addition, when a retinal pigment epithelial cell sheet is transplanted, as a transplantation material for disease treatment of the present invention, under retina, a transplantation method including placing the sheet on a water flow from an injection needle inserted into the transplantation site under retina of an eye, or an exclusive treatment apparatus for transportation may be used.

The present invention further provides Bruch's membrane obtained by the aforementioned method for producing Bruch's membrane. Since the Bruch's membrane of the present invention is a cell-free basement membrane, it is under less regulatory restraints as compared to cell transplantation and transplantation of Bruch's membrane has a lower threshold for practicalization. In addition, it is also useful for research use such as functional analysis and the like.

### Examples

The present invention is explained in more detail in the following by referring to Examples. However, they are mere exemplifications, and do not limit the scope of the present invention in any manner.

### Preparation of human retinal pigment epithelial cells from an eye

Human retinal pigment epithelial (RPE) cells (hRPECs) were collected from several donors within 48 hr after death. The cells were suspended in Ham F-10 medium (10% fetal bovine serum, medium for diagnosis (Glutamax II; Invitrogen) penicillin G (100 IU/mL), streptomycin (0.1 mg/mL) and amphotericin B (0.25 mg/L)), and seeded on a 10 cm dish. The cells were cultured under an atmosphere of 95% air/5% CO₂ at 37°C.

### Consideration of cell number for seeding

Before cell seeding, the number of cells to be seeded was considered. The diameter of one cell of retinal pigment epithelial cells is 10 - 20 µm. In the present Example, it was assumed 10 µm, and the area of one cell was estimated to be 5x5x3.14=78.5 µm². Therefore, when 1 mm² is to be occupied with cells, the number of cells to be seeded is 1,000×1,000/78.5=about 12,740 cells. In the present Example, 4 well Chamber Slide (Lab-Tek) (2 cm² per well) was used as a plane substrate. To occupy 1 well with cells, 12,740×2×10×10=2,548,000, i.e., about 2,500,000 cells, could be estimated to be necessary. In normal cell culture, the number of retinal pigment epithelial cells grown to confluence in a 75 cm² culture dish is about 1,000,000 - 2,500,000 cells and, assuming 2,500,000 cells, the cell density is 2,500,000 cells/75 cm²=about 333 cells/mm². The number is considerably less than the cell density in the body (about 4,000 cells/mm²) and the cell density employed in the present culture method (for example, 4,000,000 cells, cell density 20,000 cells/mm²).

### Example 1

### Formation of human retinal pigment epithelial sheet

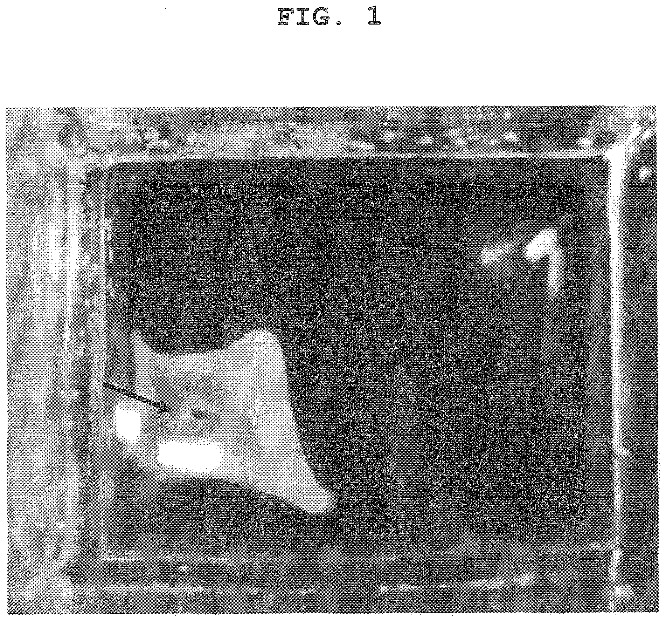

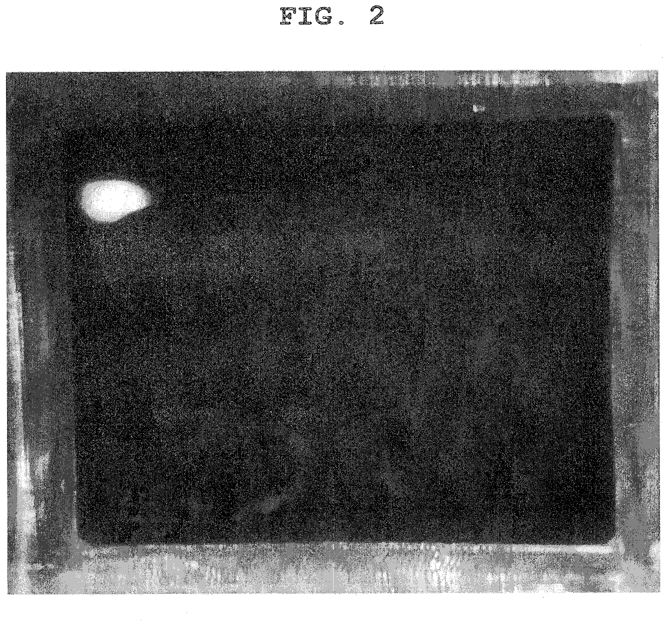

Retinal pigment epithelial cells collected from an eye were cultured, treated with 0.1% trypsin and 0.02% EDTA for 5 min, and collected by pipetting. The collected RPE cells were centrifuged, the number thereof was counted, and the cells were seeded by 1,000,000 cells and 2,000,000 cells on 4 well Chamber Slide (Lab-Tek) (5,000 cells/mm², 10,000 cells/mm², respectively), according to the above-mentioned results of consideration. As a result, a sheet was formed, but shrank to 1/4 and 1/2 of the well area, respectively (Fig. 1). Assuming the number of cells without shrinkage was 2,000,000 cells/cm², the cells were freshly seeded on 4 well Chamber Slide. As a result, shrinkage of the cell sheet was not observed (Fig. 2).

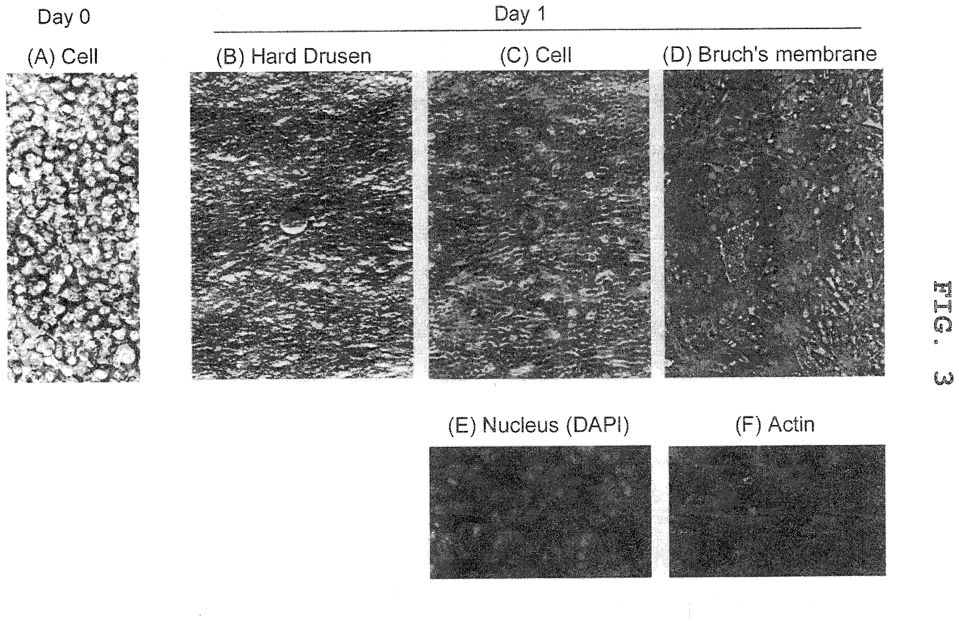

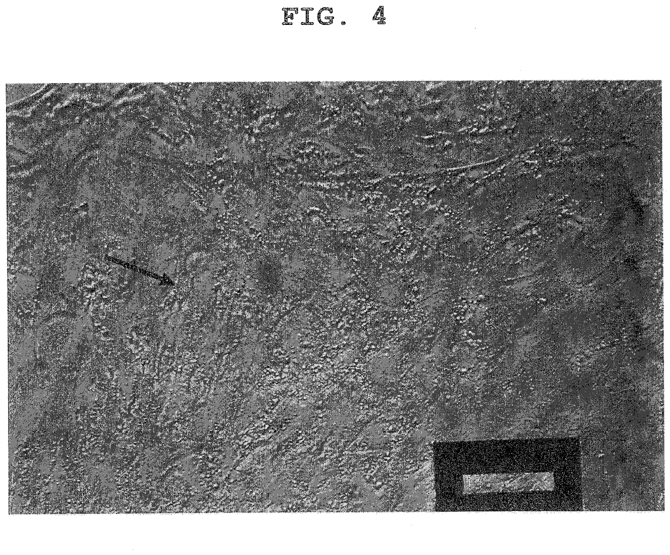

In addition, the cell sheet was immunostained. For immunostaining, the sheet was fixed with 4% paraformaldehyde and whole mount was performed, or a cryo or paraffin section was prepared, and stained by an enzyme antibody method or a fluorescence antibody method by using a primary antibody to an object protein such as elastin, collagen IV, collagen I, actin, cytokeratin, occludin, ZO-1 and the like, and observed under an optical microscope or a fluorescence microscope. As for actin, it can also be stained with fluorescence-labeled phalloidine and is observable. As a result of such immunostaining, cell-cell adhesion and expression of smooth muscle actin was confirmed 1 day after the seeding (Fig. 3). Thereafter, expression of elastin and collagen IV, which are the components of Bruch's membrane, was confirmed on the serum contact surface of RPE cells (Fig. 4). No report has documented heretofore that expression of elastin could be confirmed in an in vitro culture system of retinal pigment epithelial cells.

In addition, similar results as in the aforementioned immunostaining could also be obtained by separately performing as mentioned below.

A human retinal pigment epithelial sheet produced was washed twice with 1M phosphate buffered saline (PBS), and fixed in 0.2M PBS containing 4% paraformaldehyde for 15 min. After fixation, the retinal pigment epithelial sheet was washed 5 times with Tween 20-containing Tris-buffer saline (TBS-T). Then, the cell sheet was blocked with a protein blocking buffer and cultured with a primary antibody at 4°C overnight. As the primary antibody, a goat polyclonal anti-occludin antibody (1:100; Santa Cruz Biotechnology) and rabbit polyclonal anti-elastin antibody (1:100; Calbiochem) were used. The cell sheet was washed 3 times with TBS-T and cultured with a secondary antibody at room temperature for 30 min. Then, the cell sheet was washed 3 times with TBS-T, dried, and mounted with vectashield together with DAPI. Each piece was observed with an optical microscope and a fluorescence microscope (AX-70; Olympus optical Co., Ltd., Tokyo, Japan).

### Example 2

### Formation of human iPS cell-derived human retinal pigment epithelial sheet

In the same manner as in Example 1 except that iPS cell-derived RPE cells were used as the human retinal pigment epithelial cells, a human retinal pigment epithelial sheet was formed. As the iPS cell-derived RPE cells, RPE cells obtained by differentiation induction of human iPS cells, which were obtained by the method described in Neuroscience Letters 458 (2009) 126-131, according to the method described in WO01/088100, were used.

### Example 3

### Western blotting of human retinal pigment epithelial sheet-derived protein

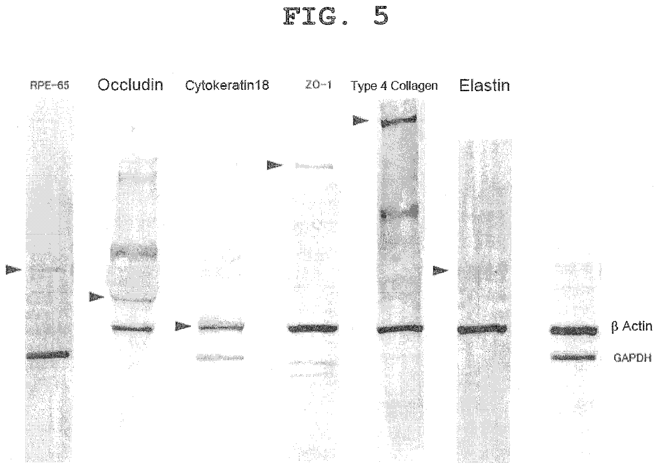

An extract of the human retinal pigment epithelial sheet produced in Example 1 was lysed with a lysis buffer containing a protease inhibitor. The equal amounts of the obtained protein was separated by SDS-PAGE (10%), transferred onto a polyvinylidene difluoride membrane (Immobilon-P; Millipore, Billerica, MA), and blocked with 10% skim milk dissolved in TBS-T for 1 hr. Then, the membrane was incubated overnight at 4°C with a primary antibody obtained by dissolving each of a mouse monoclonal anti-RPE-65 antibody (1:5000), a rabbit polyclonal anti-occludin antibody (1:250), a mouse monoclonal anti-cytokeratin 18 antibody (1:1000), a rabbit polyclonal anti-ZO-1 antibody (1:50), a mouse monoclonal anti-type 4 collagen antibody (1:1000), and a mouse monoclonal anti-elastin antibody (1:1000) in 1% skim milk-containing TBS-T. After washing 6 times with TBS-T, the membrane was incubated with a secondary antibody (50 ng/ml) conjugated with alkaline phosphatase for 1 hr. The membrane was washed 6 times with a wash buffer to remove the antibody, and stained with a chemical fluorescence buffer (Fig. 5). For standardization, the membrane was reprobed with a rabbit monoclonal anti-GAPDH antibody or rabbit monoclonal anti-actin antibody. Expression of a protein to each of the above-mentioned antibodies was observed in the protein derived from the human retinal pigment epithelial sheet.

### Example 4

### Electron microscopic image analysis of human retinal pigment epithelial sheet

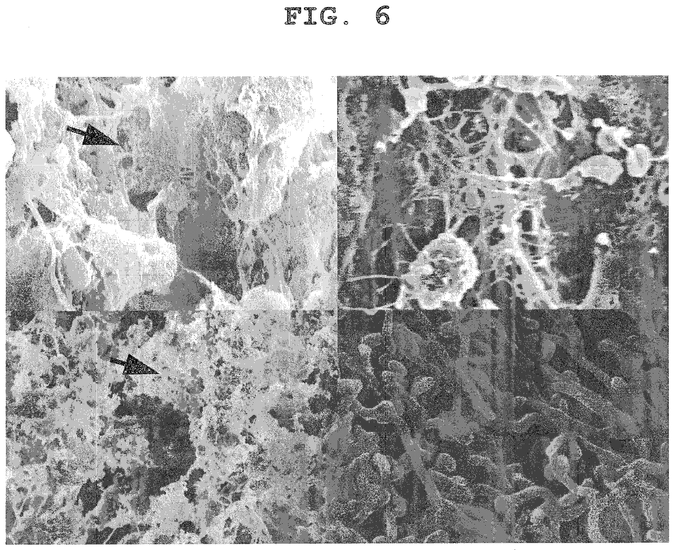

The human retinal pigment epithelial sheet produced in Example 1 was recovered, and fixed in a 2.5% glutaraldehyde-containing 0.1M phosphate buffer (PB) for 60 min. After washing with 0.1M PBS, the retinal pigment epithelial sheet was left standing in 1% OsO₄-containing 0.1M PBS for 2 hr. After fixation, the sheet was dehydrated with ethanol and t-butylalcohol, and dried. Then, the retinal pigment epithelial sheet was subjected to sputter coating (distance to target sample 50 mm, at 5×10⁻² milli bar 30 mA, for 40 seconds) with palladium, and observed with a scanning electron microscope (Fig. 6). Formation of collagen fiber and elastic fiber (Fig. 6, arrow) was observed in electron microscopic images.

### Industrial Applicability

According to the method of the present invention, a retinal pigment epithelial cell sheet for transplantation application to patients with age-related macular degeneration can be prepared relatively conveniently. Particularly, when the cells to be used for culture are iPS cell-derived retinal pigment epithelial cells, rejection in transplantation can be avoided since the patients' own cells can be utilized.

This application is based on patent application No. 2010-108674 (filing date: May 10, 2010) filed in Japan.

## Claims

1. A method for producing a cell sheet, comprising the following steps;
(1) a step of preparing a retinal pigment epithelial cell,
(2) a step of seeding the prepared cell on a plane substrate at 10,000 cells/mm² - 120,000 cells/mm², and culturing the cell, wherein the cell sheet comprises a basement membrane formed on the side opposite from the side to be in contact with the plane substrate, and
(3) a step of confirming formation of Bruch's membrane on the side opposite from the side to be in contact with the plane substrate of the cell.

2. The method according to claim 1, wherein the obtained cell sheet comprises a tight junction formed between cells.

3. The method according to claim 1, further comprising the following step (4):
(4) a step of confirming the presence or absence of expression of a differentiation marker in the cultured cells obtained in step (2).

4. The method according to claim 3, wherein, in step (4), the differentiation marker is at least one kind selected from the group consisting of bestrophin-1, RPE-65, cytokeratin, occludin, ZO-1, elastin, actin, type 1 collagen and type 4 collagen.

5. A method for producing Bruch's membrane comprising a step of separating the Bruch's membrane formed by the method according to any one of claims 1 to 4.

6. A cell sheet produced by the method according to any one of claims 1 to 4.

7. A material for transplantation for use in treating a disease, comprising a cell sheet produced by the method according to any one of claims 1 to 4.

8. Bruch's membrane produced by the method according to claim 5.

## Patentansprüche

1. Verfahren zur Herstellung einer Zellplatte, das die folgenden Schritte umfasst:
(1) einen Schritt des Herstellens einer retinalen Pigmentepithelzelle;
(2) einen Schritt des Aussäens der hergestellten Zelle auf einem planaren Substrat mit 10 000 Zellen/mm² bis 120 000 Zellen/mm² und Kultivieren der Zelle, wobei die Zellplatte eine Basalmembran umfasst, die auf der Seite gegenüber von der Seite, die mit dem planaren Substrat in Kontakt sein soll, gebildet ist; und
(3) einen Schritt des Bestätigens der Bildung einer Bruch-Membran auf der Seite gegenüber von der Seite, die mit dem planaren Substrat der Zelle in Kontakt sein soll.

2. Verfahren gemäß Anspruch 1, wobei die erhaltene Zellplatte eine Tight Junction umfasst, die zwischen Zellen entstanden ist.

3. Verfahren gemäß Anspruch 1, das weiterhin den folgenden Schritt (4) umfasst:
(4) einen Schritt des Bestätigens der Anwesenheit oder Abwesenheit der Expression eines Differenzierungsmarkers in den in Schritt (2) erhaltenen kultivierten Zellen.

4. Verfahren gemäß Anspruch 3, wobei es sich bei dem Differenzierungsmarker in Schritt (4) um wenigstens eine Art handelt, die aus der Gruppe ausgewählt ist, die aus Bestrophin-1, RPE-65, Cytokeratin, Occludin, ZO-1, Elastin, Actin, Typ-1-Collagen und Typ-4-Collagen besteht.

5. Verfahren zur Herstellung einer Bruch-Membran, umfassend einen Schritt des Abtrennens der nach dem Verfahren gemäß einem der Ansprüche 1 bis 4 gebildeten Bruch-Membran.

6. Zellplatte, hergestellt nach dem Verfahren gemäß einem der Ansprüche 1 bis 4.

7. Material zur Transplantation zur Verwendung bei der Behandlung einer Krankheit, umfassend eine Zellplatte, die nach dem Verfahren gemäß einem der Ansprüche 1 bis 4 hergestellt ist.

8. Bruch-Membran, hergestellt nach dem Verfahren gemäß Anspruch 5.

## Revendications

1. Méthode de production d'un feuillet cellulaire, comprenant les étapes suivantes ;
(1) une étape de préparation d'une cellule épithéliale pigmentaire rétinienne,
(2) une étape d'ensemencement de la cellule préparée sur un substrat plan à hauteur de 10 000 cellules/mm² à 120 000 cellules/mm², et de culture de la cellule, dans laquelle le feuillet cellulaire comprend une membrane basale formée sur le côté opposé au côté qui doit se trouver en contact avec le substrat plan, et
(3) une étape de confirmation de la formation de la membrane de Bruch sur le côté opposé au côté qui doit se trouver en contact avec le substrat plan de la cellule.

2. Méthode selon la revendication 1, dans laquelle le feuillet cellulaire obtenu comprend une jonction serrée formée entre les cellules.

3. Méthode selon la revendication 1, comprenant en outre l'étape (4) suivante :
(4) une étape de confirmation de la présence ou de l'absence d'expression d'un marqueur de différenciation dans les cellules cultivées obtenues à l'étape (2).

4. Méthode selon la revendication 3, dans laquelle, à l'étape (4), le marqueur de différenciation est au moins une sorte sélectionnée dans le groupe constitué par la bestrophine-1, RPE-65, la cytokératine, l'occludine, ZO-1, l'élastine, l'actine, le collagène de type 1 et le collagène de type 4.

5. Méthode de production d'une membrane de Bruch comprenant une étape de séparation de la membrane de Bruch formée par la méthode selon l'une quelconque des revendications 1 à 4.

6. Feuillet cellulaire produit par la méthode selon l'une quelconque des revendications 1 à 4.

7. Matériau de transplantation pour son utilisation dans le traitement d'une maladie, comprenant un feuillet cellulaire produit par la méthode selon l'une quelconque des revendications 1 à 4.

8. Membrane de Bruch produite par la méthode selon la revendication 5.
